# EUROPÄISCHE PATENTSCHRIFT

(11) **EP 1 584 350 B1**
(45) Veröffentlichungstag und Bekanntmachung des Hinweises auf die Patenterteilung: **04.01.2012**
(21) Anmeldenummer: 05102602.9
(22) Anmeldetag: 01.04.2005
(51) Int. Cl.: A61N 1/375, H01R 13/18, H01R 13/187

(54) **Federkontaktelement**
Spring contact member
Elément de contact de ressort

(30) Priorität: 05.04.2004 DE 102004017659
(43) Veröffentlichungstag der Anmeldung: 12.10.2005
(60) Teilanmeldung: 08008087.2
(73) Patentinhaber: Biotronik CRM Patent AG, 6340 Baar (CH)
(72) Erfinder: Olbertz, Ralf, 12347, Berlin (DE)
(74) Vertreter: Lindner-Vogt, Karin L.

(56) Entgegenhaltungen:
- EP-A- 0 590 756
- EP-A- 0 840 400
- EP-A- 0 848 584
- US-A- 5 275 620
- US-A- 5 730 628
- US-A1- 2004 167 582

## Beschreibung

Die Erfindung betrifft eine Kontakt-Anordnung zum Anschluss einer Elektrodenleitung an ein implantierbares Gerät. Die Kontakt-Anordnung weist eine elektrische Anschlussbuchse mit einer Buchsenlängsachse und wenigstens einer Öffnung zum Einführen eines Steckers entlang der Buchsenlängsachse auf. Die Kontakt-Anordnung weist auch mindestens ein elektrisch leitfähiges Federkontaktelement auf, welches ausgebildet und angeordnet ist, beim Einführen eines Steckers federnd elastisch nach außen auszuweichen, dabei wenigstens in einer quer zur Buchsenlängsachse verlaufenden Federauslenkungsrichtung komprimiert zu werden und eine dem Komprimieren entgegenwirkende Federkraft auszubilden.

Feder-Kontaktelemente für implantierbare Geräte sind aus dem Stand der Technik bekannt. Aus der DE 196 09 367 der Anmelderin ist ein Herzschrittmacher mit einer rund ausgebildeten Hülse zur Aufnahme einer Elektrodenleitung bekannt, welche radial nach innen federnde Feder-Kontaktelemente aufweist, welche gleichmäßig am Hülsenumfang verteilt sind. Die Feder-Kontaktelemente sind streifenförmig ausgebildet, einseitig an der Hülsenwand befestigt und weisen in den Hülsenraum, wobei ihr freies Ende halbkugelförmig konvex ausgebildet ist und mit der konvexen Seite radial nach innen weist.

Solche Feder-Kontaktelemente haben das Problem, dass sich die Kontaktfeder-Andruckkraft nur schwierig einstellen lässt, insbesondere, wenn - wie oft üblich - das Federelement aus der Hülsenwand ausgestanzt und die somit erhaltene Blattfeder in den Innenraum gebogen ist. Hinsichtlich der Materialauswahl zur Herstellung der Feder ist man hier auf das Hülsenmaterial angewiesen, so dass in einem solchen Fall stets ein Kompromiss zwischen Anforderungen an eine Hülse und den Anforderungen an eine Kontaktfeder zur Herstellung eines sicheren und guten elektrischen Wirkkontaktes mit einer Elektrodenleitung gebildet wird.

Die der Erfindung zugrunde liegende Aufgabe ist es daher, ein Kontaktelement anzugeben, welches einen sicheren galvanischen Kontakt mit einem Elektrodenleitungs-Stecker ermöglicht.

Dokument EP-A-0 840 400 offenbart eine Vorrichtung gemäß Oberbegriff des Anspruchs 1.

Die Aufgabe wird durch eine Kontakt-Anordnung mit einer Anschlussbuchse für ein implantierbares Gerätes der eingangs genannten Art gelöst, wobei das FederKontaktelement das Feder-Kontaktelement eine einstückige, metallische Federkontaktzunge mit quer zur Federauslenkungsrichtung verlaufenden Federkontaktzungen-Abschnitten aufweist, die durch Wendeabschnitte miteinander verbunden sind, wobei in Federrichtung unmittelbar aufeinanderfolgende Wendeabschnitte jeweils einen entgegengesetztem Wenderichtungssinn aufweisen, wobei das Feder-Kontaktelement drei oder vier Wendeabschnitte aufweist.

In einer bevorzugten Ausführungsform weist das Feder-Kontaktelement eine Federkontaktzunge auf. Die Federkontaktzunge ist derart gewendet ist, dass die Federkontaktzunge in einer Aufsicht auf eine von der Buchsenlängsachse und der Federauslenkungsrichtung aufgespannten Ebene eine Wellenform hat.

Bei einem wellenförmigen Feder-Kontaktelement mit zwei oder mehreren Wendeabschnitten lässt sich besonders vorteilhaft ein Feder-Kontaktelement mit einer weichen Federsteife in Federauslenkungsrichtung ausbilden. Ein weiterer Vorteil eines solchen Feder-Kontaktelements ist, dass die Federsteife in Federrichtung leicht einstellbar ist.

Die Wellenform des Feder-Kontaktelements kann eine S-förmige, eine rechteckförmige oder eine dreieckförmige Wellenform sein. Durch die Ausbildung des Federkontaktelements gemäß verschiedener Wellenformen weist das Feder-Kontaktelement je nach gewählter Wellenform eine unterschiedliche Federsteife, insbesondere in Abhängigkeit von einer durch das Komprimieren bewirkten Federauslenkung auf. Dadurch lässt sich vorteilhaft eine gewünschte Federsteife über die Wahl der Wellenform einstellen.

Durch die erzielte Serienschaltung von - jeweils durch Wendeabschnitte gebildete - Feder-Kontaktelemente kann ein Feder-Kontaktelement eine besonders weiche Federsteife aufweisen.

In einer bevorzugten Ausführungsform ist die Federkontaktzunge des Feder-Kontaktelements eine gewendete Blattfeder. Die Blattfeder hat ein Blattfeder-Querschnittsprofil, dessen Breitenabmessung größer ist als dessen Höhenabmessung. Durch die Ausbildung als gewendete Blattfeder weist das Feder-Kontaktelement eine hohe Torsionsstabilität tangential zur Federauslenkungsrichtung auf. Im Anwendungsfall lässt sich ein Feder-Kontaktelement nicht so leicht verbiegen, wenn ein Elektrodenleitungs-Stecker nicht genau parallel zur Buchsenlängsachse in die Elektrodenleitungs-Anschlussbuchse eingeführt wird.

Bevorzugt beträgt die Breitenabmessung des Blattfederquerschnitts das fünffache der Höhenabmessung des Blattfederquerschnitts.

In einer alternativen Ausführungsform kann die Federkontaktzunge einen quadratischen oder runden Querschnitt aufweisen.

In einer bevorzugten Ausführungsform weist die Anordnung mindestens drei Feder-Kontaktelemente auf, wobei die Federauslenkungsrichtungen der Feder-Kontaktelemente in einer gemeinsamen, senkrecht zur Buchsenlängsachse verlaufenden Ebene angeordnet sind.

Dadurch kann ein Ringkontakt eines Elektrodenleitungs-Steckers im Anwendungsfall von mehreren Feder-Kontaktelementen berührt werden, so dass dadurch eine hohe Kontaktsicherheit, verbunden mit einem niedrigen elektrischen Kontakt-Übergangswiderstand erzielt wird.

In einer bevorzugten Ausführungsform weist ein Kontaktabschnitt der Federkontaktzunge einen zur Berührung mit einem Gegenkontakt eines Elektrodenleitungs-Steckers vorgesehenen Oberflächenbereich auf, welcher konvex geformt ist. Im Anwendungsfall wird dadurch vorteilhaft das Einführen eines Elektrodenleitungs-Steckers in die Elektrodenleitungs-Anschlussbuchse erleichtert.

Vorteilhafte Ausführungsformen einer Federkontaktzunge weisen eines oder eine Kombination der folgenden Materialien auf:

Hochlegierte Stähle nach DIN 17224: X 12 CrNi 17 7; X 7 CrNiAl 17 7; oder X 5 CrNiMo 18 10 oder andere hochlegierte Metallegierungen.

Eine Federkontaktzunge kann wenigstens abschnittweise vorteilhaft versilbert, vergoldet, mit Platinbromid (PtBr) beschichtet oder eine Kombination aus diesen Beschichtungen aufweisen. Beispielsweise ist eine Federkontaktzunge verkupfert und obenauf versilbert.

In einer anderen Ausführungsform weist das Feder-Kontaktelement wenigstens ein Elastomer-Element auf, welches einen Zwischenraum mindestens teilweise auffüllt, welches sich zwischen zwei in Federauslenkungsrichtung gegenüberliegenden Blattfeder-Abschnitten erstreckt, wobei das Elastomer-Material als Auslenkungsweg-Begrenzer ausgebildet und angeordnet ist.

Dadurch wird vorteilhaft eine plastische Verformung des Feder-Kontaktelements, verursacht durch eine zu große Auslenkung, verhindert. Beispielsweise ist das Elastomer-Element ein Silikon-Elastomer-Element, welches einen Silikonkautschuk enthält.

In einer bevorzugten Ausführungsform ist das Elastomer-Element derart ausgeformt, dass es den Zwischenraum teilweise, bevorzugt bis zur Hälfte in Auslenkungsrichtung auffüllt.

In einer anderen Ausführungsform ist das Elastomer-Element ausgeformt, den Zwischenraum vollständig aufzufüllen, wobei das Elastomer-Element-Material einen Elastizitätsmodul aufweist, welcher derart gewählt ist, dass das Elastomer-Element die Gesamtfedersteife des Feder-Kontaktelements entlang der Feder-auslenkungsrichtung nicht wesentlich erhöht.

Bevorzugt ist das Elastizitätsmodul des Elastomer-Elements derart gewählt, dass die Gesamtfedersteife des Feder-Kontaktelements um nicht mehr als 5 % erhöht ist im Vergleich zu einer Federsteife eines Feder-Kontaktelements ohne ein Elastomer-Element.

In einer vorteilhaften Ausführungsform ist das Elastomer-Element derart ausgeformt, dass es das Feder-Kontaktelement bis auf einen zum elektrischen Kontaktieren vorgesehenen Abschnitt vollständig umgibt. Bei dieser Ausführungsform ist das Feder-Kontaktelement vorteilhaft gegen Feuchtigkeit, Korrosion und plastische Verformung geschützt.

In einer bevorzugten Ausführungsform weist eine Feder-Kontaktelement-Anordnung für eine Kontakt-Anordnung einen Feder-Kontaktelement-Träger mit mindestens drei, bevorzugt vier, weiter bevorzugt sechs angeformten Feder-Kontaktelementen auf. Der Feder-Kontaktelement-Träger ist als kreisrunde flache Scheibe ausgebildet, welche einen - bevorzugt zentrisch angeordneten - Durchbruch zur Durchführung eines Steckers aufweist.

In einer weiter bevorzugten Ausführungsform sind die Feder-Kontaktelemente am Feder-Kontaktelement-Träger in Umfangsrichtung gleichmäßig angeordnet und im Bereich des äußeren Randes des Feder-Kontaktelement-Trägers angeformt.

Besonders bevorzugt sind die Feder-Kontaktelemente jeweils aus einer wellenförmig gewendeten Blattfederzunge gebildet, wobei die Blattfederzunge zwei Wendeabschnitte mit jeweils einander entgegengesetztem Wenderichtungssinn aufweist und so eine S-förmige Wellenform hat. Die Blattfederzungen sind ausgebildet, unter Auslenkung entlang einer Federauslenkungsachse eine der Auslenkung entgegenwirkende Federkraft auszubilden und beim Einführen eines Steckers federnd elastisch nach außen auszuweichen.

In einer vorteilhaften Ausführungsvariante weist der Feder-Kontaktelement-Träger in Umfangsrichtung jeweils seitlich an die Federkontaktelemente anschließende Aussparungs-Bereiche aufweist.

Weiter bevorzugt weist der Feder-Kontaktelement-Träger in Umfangsrichtung an der Innenkante, welche an den Durchbruch des Feder-Kontaktelement-Trägers angrenzt, jeweils gegenüber eines jeden Federkontaktelements angeordnete Aussparungsbereiche auf. Die Aussparungen am inneren Rand des Feder-Kontaktelement-Trägers zum Durchbruch hin sind bevorzugt derart tief in radiale Richtung nach außen eingeschnitten, dass ein noch stehender Bereich des Feder-Kontaktelement-Trägers zwischen den äußeren und den inneren Aussparungen - gesehen in radialer Richtung - eine in tangentialer Richtung wirkende Torsionsfeder bildet. Die Torsionsfeder wirkt in Serie zu den Federkontaktelementen und erleichtert vorteilhaft das Einführen eines Steckers in eine Anschlussbuchse.

In einer bevorzugten Ausführungsvariante ist der Feder-Kontaktelement-Träger mit den Federkontaktelementen aus einem einstückigen Blech gebildet, welches strahlenförmig radial nach außen weisende Blattfederstreifen aufweist. Die Feder-Kontaktelemente sind jeweils aus den strahlenförmig radial nach außen weisenden Blattfederstreifen gebildet, wobei die Blattfederstreifen am Umfangsansatz des Feder-Kontaktelement-Trägers - bevorzugt rechtwinklig - abgewinkelt und im weiteren Verlauf zu einer S-förmigen Wellenform ausgeformt sind.

Der Feder-Kontaktelement-Träger mit den Federkontaktelementen kann aus einem Blech gestanzt oder lasergeschnitten sein.

In einer vorteilhaften Ausführungsform sind die Aussparungen seitlich der Feder-kontaktelemente derart tief in radiale Richtung eingeschnitten, dass die Aussparungen seitlich der Federkontaktelemente derart tief in radiale Richtung eingeschnitten sind, dass nach mindestens rechtwinkligem Abwinkeln eines Blattfederstreifens ein Abschnitt des Blattfederstreifens beginnend am Umfang des Feder-Kontaktelement-Trägers (215) einen äußeren Umfangsradius des Feder-Kontaktelement-Trägers (215) neben den Aussparungen nicht überschreitet.

In einer bevorzugten Ausführungsform umfasst die Anordnung auch eine Hülse mit einer Hülsenlängsachse und einer Hülsenwand. Die Hülsenwand umschließt einen Hülseninnenraum, welcher wenigstens eine Öffnung zum Einführen eines Elektroden-Steckers in Richtung der Hülsenlängsachse aufweist.

In der hier beschriebenen Ausführungsform sind die Hülsenlängsachse und die Buchsenlängsachse der Anschlussbuchse zueinander parallel oder bilden eine gemeinsame Achse. Das Feder-Kontaktelement kann wenigstens teilweise in dem Hülseninnenraum angeordnet sein.

In einer Ausführungsform weist die Hülsenwand einen Durchbruch zur Aufnahme eines Feder-Kontaktelements auf, wobei das Feder-Kontaktelement derart in dem Durchbruch angeordnet ist, dass mindestens ein zum elektrischen Kontaktieren eines Elektrodenleitungs-Steckers vorgesehener Abschnitt des Feder-Kontaktelements in dem Hülseninnenraum angeordnet ist.

In einer besonders bevorzugten Ausführungsform ist die Hülsenwand zylindrisch ausgebildet und der Hülseninnenraum weist in Richtung der Hülsenlängsachse zwei Öffnungen zur Aufnahme eines Elektroden-Steckers auf, so dass die Hülse in Richtung der Hülsenlängsachse vollständig durchbrochen ist. Die Hülsenwand kann auch einen halbkreisförmigen, rechteckigen oder quadratischen Querschnitt haben.

Weiter bevorzugt weist die Hülse mindestens drei, besonders bevorzugt sechs Feder-Kontaktelemente auf, wobei die Feder-Kontaktelemente in einer gemeinsamen, senkrecht zur Buchsenlängsachse verlaufenden Ebene angeordnet sind.

Die Erfindung betrifft auch ein implantierbares Gerät mit einer zuvor beschriebenen Kontakt-Anordnung. Das implantierbare Gerät kann ein Herzschrittmacher, ein Kardioverter, ein Defibrillator oder eine Kombination aus diesen sein. Die Elektrodenleitungs-Anschlussbuchse ist bevorzugt im Header eines implantierbaren Gerätes angeordnet.

Die in den Figuren gezeigten Ansführungs beispiele sind nicht Teil der Erfindung.

Figur 1 zeigt schematisch einen Längsschnitt eines Ausführungsbeispiels einer Anordnung für eine Elektrodenleitungs-Anschlussbuchse mit einer Hülse und Feder-Kontaktelementen.

Figur 2a zeigt schematisch einen Längsschnitt einer Anordnung mit einer Hülse, welche Feder-Kontaktelemente und eine Dichtung aufweist.

Figur 2b zeigt schematisch eine Explosionsdarstellung der in Figur 2 in Längsschnitt gezeigten Anordnung.

Figuren 3a und b zeigen eine Alternative zur der in Figur 2 abgebildeten Ausführungsvariante.

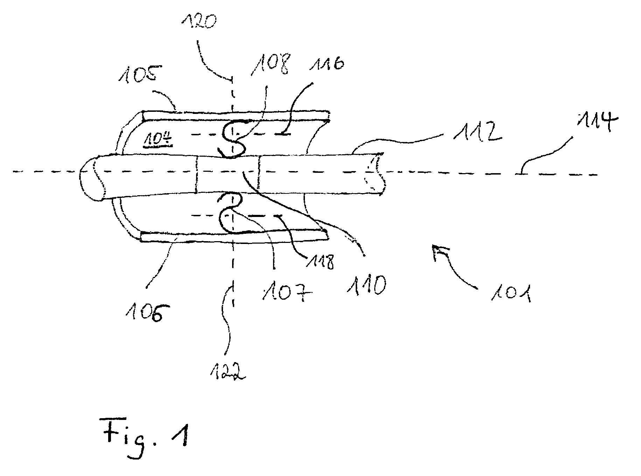

Figur 1 zeigt - schematisch dargestellt - einen Längsschnitt einer Anordnung 101 für eine Elektrodenteitungs-Anschlussbuchse eines implantierbaren Geräts.

Die Anordnung 101 umfasst eine Hülse 105 mit einer zentrisch verlaufenden Hülsenlängsachse 114, wobei die Hülse 105 einen Hülseninnenraum 104 umschließt.

Die Hülse 105 kann in einer Elektrodenleitungsbuchse derart angeordnet sein, dass die Hülsenlängsachse 114 mit einer Buchsenlängsachse eine gemeinsame Achse bildet oder zu dieser parallel ist.

Der Hülseninnenraum 104 ist an den Enden der Hülse 105 jeweils geöffnet, so dass ein - in dieser Figur abschnittsweise dargestellter - Stecker einer Elektrodenleitung 112 entlang der Hülsenlängsachse 114 durchgängig in den Hülseninnenraum 104 eingeführt werden kann.

Die Hülse 105 umfasst eine Hülsenwand 106, wobei an der Innenseite der Hülsenwänd 106 Feder-Kontaktelemente 107 und 108 in radialer Richtung nach innen weisend angeordnet sind. Die Feder-Kontaktelemente 108 und 107 sind in dem Hülseninnenraum angeordnet, in radialer Richtung der Hülsenlängsachse 114 und somit auch dem in dieser Abbildung dargestellten eingeführten Stecker einer Elektrodenleitung 112 entgegenzufedern.

Das Feder-Kontaktelement 107 ist aus einer metallischen Federzunge gebildet und parallel zu einer Federquerachse 118 alternierend entgegengesetzt gewendet, so dass das Feder-Kontaktelement 107 zwei Wendeabschnitte mit jeweils entgegengesetztem Wenderichtungssinn aufweist. Das Feder-Kontaktelement 107 ist ausgebildet, unter Auslenkung wenigstens entlang einer FederAuslenkungsachse 122 eine der Auslenkung entgegenwirkende Kraft auszubilden. Die Auslenkung wird in diesem Ausführungsbeispiel durch den entlang der Hülsenlängsachse 114 eingeführten Stecker einer Elektrodenleitung 112 entlang der Federauslenkungsachse 122 radial nach außen, also in Richtung Hülsenwand 106 bewirkt, so dass das Feder-Kontaktelement 107 komprimiert wird.

Die Hülse 105 umfasst auch ein an der Innenseite der Hülsenwand 106 angeordnetes Feder-Kontaktelement 108.

Das Feder-Kontaktelement 108 ist genauso wie das Feder-Kontaktelement 107 aus einer metallischen Federzunge gebildet und weist auch zwei Wendeabschnitte mit jeweils entgegengesetztem Wenderichtungssinn auf.

Das Feder-Kontaktelement 108 ist ausgebildet, unter Auslenkung entlang einer Federauslenkungsachse 120 eine der Auslenkung entgegenwirkende Federkraft auszubilden. Die Federauslenkungsachse 120 und die Federquerachse 116 sind zueinander orthogonal; die Federauslenkungsachse 122 und die Federquerachse 116 sind auch zueinander orthogonal.

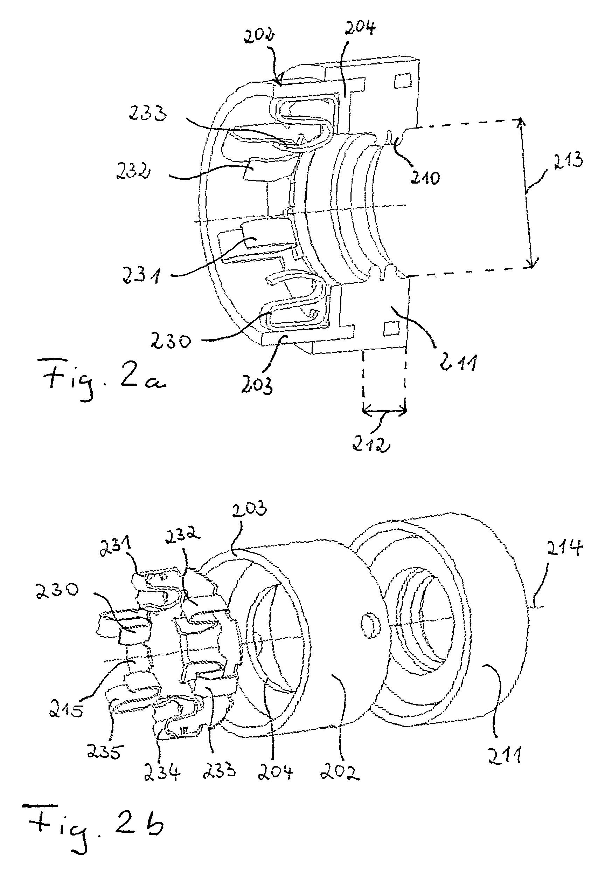

Figur 2a zeigt - schematisch dargestellt - einen Längsschnitt einer Darstellung einer Anordnung 201 für eine Elektrodenleitungs-Anschlussbuchse, welche eine Hülse 202 mit einer Hülsenwand 203, Feder-Kontaktelemente 230, 231, 232, 233, und einen Dichtungsring 211 mit einer Dichtungsringdicke 212 umfasst. Der Dichtungsring 211 weist einen kreisrunden Durchbruch 213 zur Aufnahme einer Elektrodenleitung mit rundem Querschnitt auf und ist derart geformt, dass die Hülsenwand gegen den Durchbruch 213 abgedichtet ist.

Die Hülse 202 weist eine in dem Hülseninnenraum angeordnete Trennwand 204 auf, welche den Hülseninnenraum entlang einer - in dieser Abbildung nicht dargestellten - Hülsenlängsachse teilt. Die Trennwand 204 weist einen zentrisch angeordneten kreisrunden Durchbruch zum Durchführen eines Elektrodenleitungs-Steckers auf. Der Durchmesser des Trennwand- Durchbruchs entspricht dem Durchmesser des Dichtungsscheibendurchbruchs 213.

Die Dichtungsscheibe 211 weist eine Dichtungsscheibendicke 212 auf, so dass der Durchbruch der Dichtungsscheibe 211 eine Durchbruch-Innenwand aufweist. Die Durchbruch-Innenwand weist zwei in Hülsenlängsachse hintereinander angeordnete Dichtlippen auf. Die Dichtlippen weisen jeweils radial nach innen und sind an die Dichtscheibe angeformt.

Figur 2b zeigt - schematisch dargestellt - eine Explosionsdarstellung der in Figur 2a in Längsschnitt dargestellten Anordnung für eine Elektrodenleitungsbuchse.

Die Anordnung für eine Elektrodenleitungsbuchse umfasst eine Hülse 202, eine Dichtungsscheibe 211 und einen Feder-Kontaktelement-Träger 215 mit sechs angeformten Feder-Kontaktelementen 230, 231, 232, 233, 234 und 235.

Die Hülse 202 weist eine Hülsenwand 203 auf, welche einen Hülseninnenraum umfasst. Der Hülseninnenraum ist entlang der Hülsenlängsachse 214 zu den Hülsenenden wenigstens an einem Ende und im Ausführungsbeispiel an beiden Enden (Stirnseiten) geöffnet.

Die Hülse 202 weist eine Trennwand 204 auf, welche in dem Hülseninnenraum angeordnet und an die Hülsenwand 203 angeformt ist. Die Trennwand 204 weist einen zentrisch angeordneten kreisrunden Durchbruch zur Durchführung eines Elektrodenleitungssteckers entlang der Hülsenlängsachse 204 auf. Die Trennwand 204 ist von den Hülsenenden in Richtung der Hülsenlängsachse 204 jeweils beabstandet.

Der Feder-Kontaktelement-Träger 215 ist als kreisrunde flache Scheibe ausgebildet, welche einen zentrisch angeordneten Durchbruch zur Durchführung eines Elektrodenleitungssteckers aufweist.

Der Feder-Kontaktelement-Träger 215 weist sechs in Umfangsrichtung gleichmäßig angeordnete Feder-Kontaktelemente 230, 231, 232, 233, 234 und 235 auf, welche am äußeren Rand des Feder-Kontaktelement-Trägers 215 angeformt sind.

Die Feder-Kontaktelemente sind jeweils aus einer wellenförmig gewendeten Blattfeder gebildet, wobei die Blattfeder zwei Wendeabschnitte mit jeweils entgegengesetztem Wenderichtungssinn aufweist und so eine S-förmige Wellenform aufweist.

Die so ausgebildeten Feder-Kontaktelemente 230, 231, 232, 233, 234 und 235 sind ausgebildet, unter Auslenkung entlang der Federauslenkungsachse eine der Auslenkung entgegenwirkende Federkraft auszubilden und können - im Anwendungsfall - bei Einführung eines Elektrodenleitungssteckers federnd elastisch - im wesentlichen radial - nach außen ausweichen.

Der Feder-Kontaktelement-Träger 215 weist in Umfangsrichtung jeweils seitlich an die Federkontaktelemente anschließende Aussparungs-Bereiche auf.

Der Feder-Kontaktelement-Träger weist auch in Umfangsrichtung an der Innenkante, welche an den Durchbruch des Feder-Kontaktelement-Trägers angrenzt, jeweils gegenüber eines jeden Federkontaktelements angeordnete Aussparungsbereiche auf.

In diesem Ausführungsbeispiel ist der Feder-Kontaktelement-Träger 215 und die Federkontaktelemente aus einem Blechstück gestanzt. Die Feder-Kontaktelemente sind jeweils aus strahlenförmig radial nach außen weisenden Blattfederstreifen gebildet, welche zur Ausbildung der Wellenform am Umfangsansatz des Feder-Kontaktelement-Trägers 215 rechtwinklig abgebogen und im weiteren Verlauf zu einer S-förmigen Wellenform ausgeformt sind.

Die Aussparungen seitlich der Federkontaktelemente sind derart tief in radiale Richtung eingeschnitten, dass das rechtwinklig abgebogene Federkontaktelement den äußeren Durchmesser des Feder-Kontaktelement-Trägers nicht überschreitet.

Die Aussparungen am inneren Rand des Feder-Kontaktelement-Trägers 215 zum Durchbruch hin sind derart tief in radiale Richtung nach außen eingeschnitten, dass ein noch stehender Bereich des Feder-Kontaktelement-Trägers 215 zwischen den äußeren und den inneren Aussparungen - gesehen in radialer Richtung - eine in tangentialer Richtung wirkende Torsionsfeder bildet.

Die so gebildete Torsionsfeder unterstützt die Federwirkung des Feder-Kontaktelements derart, dass die Torsionsfeder in Serie zu dem Feder-Kontaktelement wirkt. Die daraus resultierende Gesamtfedersteife, gebildet aus der Federsteife der Torsionsfeder und der Federsteife des Federkontaktelements, ist kleiner als die Federsteife des Feder-Kontaktelements ohne die in Serie wirkende Torsionsfeder.

Der Feder-Kontaktelement-Träger 215 ist in seinem äußeren Umfangsdurchmesser derart bemessen, dass er entlang der Hülsenlängsachse in den Hülseninnenraum eingeführt werden kann.

Die Anordnung umfasst auch eine - in Figur 2a bereits beschriebene - Dichtungsscheibe 211, welche eine vom äußeren Dichtungsscheibenrand beabstandete Ring-Nut aufweist. Der Durchmesser der Ring-Nut entspricht dem Durchmesser der Hülse 202, sodass ein Ende der Hülsenwand 203 in Richtung der Hülsenlängsachse in die Ring-Nut der Dichtungsscheibe 212 eingeschoben werden kann.

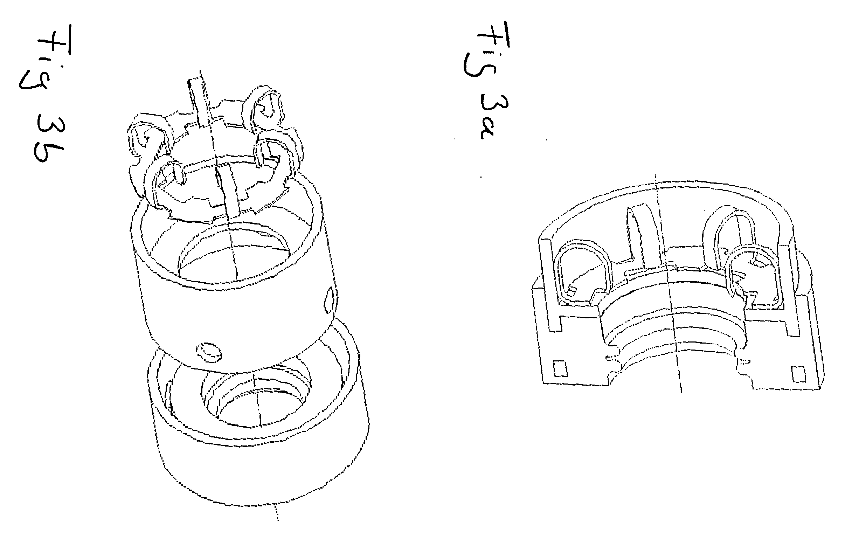

Die Ausführungsvariante gemäß Figuren 3a und 3b unterscheidet sich von der zuvor Beschriebenen durch die Gestaltung der Federkontaktelemente. Diese weisen jeweils nur einen Wendeabschnitt auf und sind C-förmig ausgebildet. Die vorteilhafte Kombination von einer Dichtscheibe mit einem Federkontaktelement-Träger mit nach innen ragenden Federkontaktelementen, die innerhalb einer gemeinsamen Hülse in Hülsenlängsrichtung hintereinander angeordnet sind, ist bei beiden Ausführungsvarianten gleichermaßen verwirklicht.

## Patentansprüche

1. Kontakt-Anordnung (101, 201) zum Anschluss einer Elektrodenleitung an ein implantierbares Gerät, mit
einer elektrischen Anschlussbuchse mit einer Buchsenlängsachse (114, 214) und wenigstens einer Öffnung zum Einführen eines Steckers entlang der Buchsenlängsachse (214),
mit mindestens einem elektrisch leitfähigen Federkontaktelement (107, 108, 230, 231, 232, 233, 234, 235), welches ausgebildet und angeordnet ist, beim Einführen eines Steckers federnd elastisch nach außen auszuweichen, dabei wenigstens in einer quer zur Buchsenlängsachse verlaufenden Federauslenkungsrichtung komprimiert zu werden und eine dem Komprimieren entgegenwirkende Federkraft auszubilden, wobei
das Feder-Kontaktelement (107, 108, 230, 231, 232, 233, 234, 235) eine einstückige, metallische Federkontaktzunge mit quer zur Federauslenkungsrichtung (120, 122) verlaufenden Federkontaktzungen-Abschnitten aufweist, die durch Wendeabschnitte miteinander verbunden sind, wobei in Federauslenkungsrichtung (120, 122) unmittelbar aufeinanderfolgende Wendeabschnitte jeweils einem entgegengesetzten Wenderichtungssinn aufweisen,
**dadurch gekennzeichnet, dass** das Feder-Kontaktelement drei oder vier Wendeabschnitte aufweist.

2. Kontakt-Anordnung (101, 201) nach Anspruch 1, **dadurch gekennzeichnet, dass** die Federkontaktzunge derart gewendet ist, dass die Federkontaktzunge in einer Aufsicht auf eine von der Buchsenlängsachse (114, 214) und der Federauslenkungsrichtung (120, 122) aufgespannten Ebene eine Wellenform hat.

3. Kontakt-Anordnung (101, 201) nach Anspruch 2, **dadurch gekennzeichnet, dass** die Wellenform eine S-förmige oder eine rechteckförmige oder eine dreieckförmige Wellenform ist.

4. Kontakt-Anordnung (101, 201) nach einem der vorhergehenden Ansprüche, **dadurch gekennzeichnet, dass** die Federkontaktzunge des Feder-Kontaktelements (107, 108, 230, 231, 232, 233, 234, 235) eine gewendete Blattfeder mit einem Blattfeder-Querschnittsprofil ist, wobei die Breitenabmessung des Blattfeder-Querschnittsprofils größer ist als die Höhenabmessung des Blattfeder-Querschnittsprofils.

5. Kontakt-Anordnung (101, 201) nach einem der vorhergehenden Ansprüche, welche mindestens drei Feder-Kontaktelemente (107, 108, 230, 231, 232, 233, 234, 235) aufweist, wobei die Federauslenkungsrichtungen der Feder-Kontaktelemente (107, 108, 230, 231, 232, 233, 234, 235) auf einer gemeinsamen, senkrecht zur Buchsenlängsachse (114, 214) verlaufenden Ebene angeordnet sind.

6. Kontakt-Anordnung (101, 201) nach einem der vorhergehenden Ansprüche, **dadurch gekennzeichnet, dass** ein Kontaktabschnitt der Federkontaktzunge einen zur Berührung mit einem Gegenkontakt eines Elektrodenleitungs-Steckers vorgesehenen Oberflächenbereich aufweist, welcher konvex geformt ist.

7. Kontakt-Anordnung (101, 201) nach einem der vorhergehenden Ansprüche, **dadurch gekennzeichnet, dass** das Feder-Kontaktelement (107, 108, 230, 231, 232, 233, 234, 235) wenigstens ein Elastomer-Element aufweist, welches einen Zwischenraum mindestens teilweise auffüllt, welcher sich zwischen zwei in radialer Richtung gegenüberliegenden Blattfederabschnitten erstreckt, wobei das Elastomer-Element als Auslenkungsweg-Begrenzer ausgebildet und angeordnet ist.

8. Kontakt-Anordnung (101, 201) nach einem der vorhergehenden Ansprüche, **dadurch gekennzeichnet, dass** die Anordnung eine Hülse (105) mit einer Hülsenlängsachse (114, 214) und einer Hülsenwand (106) aufweist, wobei
die Hülsenwand (106) einen Hülseninnenraum (104) umschließt, welcher wenigstens eine Öffnung zur Aufnahme eines Elektrodensteckers in Richtung der Hülsenlängsachse (114, 214) aufweist,
und wobei die Hülsenlängsachse (114, 214) und die Buchsenlängsachse (114, 214) zueinander parallel sind, und das Feder-Kontaktelement (107, 108, 230, 231, 232, 233, 234, 235) wenigstens teilweise in dem Hülseninnenraum (104) angeordnet ist.

9. Feder-Kontaktelement-Anordnung für eine Kontakt-Anordnung nach einem der vorhergehenden Ansprüche, mit einem Feder-Kontaktelement-Träger (215), welcher mindestens drei angeformte Feder-Kontaktelemente (230, 231, 232, 233, 234, 235) aufweist, wobei der Feder-Kontaktelement-Träger (215) als kreisförmige flache Scheibe ausgebildet ist, welche einen Durchbruch zur Durchführung eines Steckers aufweist.

10. Feder-Kontaktelement-Anordnung nach Anspruch 9; **dadurch gekennzeichnet, dass** die Feder-Kontaktelemente (230, 231, 232, 233, 234, 235) am Feder-Kontaktelement-Träger (215) in Umfangsrichtung gleichmäßig angeordnet und im Bereich des äußeren Randes des Feder-Kontaktelement-Trägers (215) angeformt sind.

11. Feder-Kontaktelement-Anordnung nach Anspruch 9 oder 10, **dadurch gekennzeichnet, dass** die Feder-Kontaktelemente (230, 231, 232, 233, 234, 235) jeweils von einer wellenförmig gewendeten Blattfederzunge gebildet sind, wobei die Blattfederzunge zwei Wendeabschnitte mit jeweils einander entgegengesetztem Wenderichtungssinn aufweist und so eine S-förmige Wellenform hat und die Blattfederzungen ausgebildet sind, unter Auslenkung entlang der Federauslenkungsachse eine der Auslenkung entgegenwirkende Federkraft auszubilden und beim Einführen eines Steckers federnd elastisch nach außen auszuweichen.

12. Feder-Kontaktelement-Anordnung nach einem der Ansprüche 9 bis 11; **dadurch gekennzeichnet, dass** der Feder-Kontaktelement-Träger (215) in Umfangsrichtung jeweils seitlich an die Federkontaktelemente anschließende Aussparungs-Bereiche aufweist.

13. Feder-Kontaktelement-Anordnung nach einem der Ansprüche 9 bis 12, **dadurch gekennzeichnet, dass** der Feder-Kontaktelement-Träger (215) in Umfangsrichtung an der Innenkante, welche an den Durchbruch des Feder-Kontaktelement-Trägers angrenzt, jeweils gegenüber eines jeden Federkontaktelements angeordnete Aussparungsbereiche aufweist.

14. Feder-Kontaktelement-Anordnung nach einem der Ansprüche 9 bis 13, **dadurch gekennzeichnet, dass** der Feder-Kontaktelement-Träger (215) mit den Federkontaktelementen aus einem einstückigen Blech gebildet ist, welches strahlenförmig radial nach außen weisende Blattfederstreifen aufweist und die Feder-Kontaktelemente jeweils aus den strahlenförmig radial nach außen weisenden Blattfederstreifen gebildet sind, wobei die Blattfederstreifen-Ansätze am Umfang des Feder-Kontaktelement-Trägers (215) abgewinkelt und im weiteren Verlauf zu einer S-förmigen Wellenform ausgeformt sind.

15. Feder-Kontaktelement-Anordnung nach Anspruch 14, **dadurch gekennzeichnet, dass** die Blattfederstreifen am Umfangsansatz des Feder-Kontaktelement-Trägers (215) rechtwinklig abgewinkelt sind.

16. Feder-Kontaktelement-Anordnung nach einem der Ansprüche 14 oder 15, **dadurch gekennzeichnet, dass** die Aussparungen seitlich der Federkontaktelemente derart tief in radiale Richtung eingeschnitten sind, dass nach mindestens rechtwinkligem Abwinkeln eines Blattfederstreifens ein Abschnitt des Blattfederstreifens beginnend am Umfang des Feder-Kontaktelement-Trägers (215) einen äußeren Umfangsradius des Feder-Kontaktelement-Trägers (215) neben den Aussparungen nicht überschreitet.

17. Implantierbares Gerät mit einer Kontakt-Anordnung (101, 201) oder einer Feder-Kontaktelement-Anordnung nach einem der vorhergehenden Ansprüche, **dadurch gekennzeichnet, dass** das implantierbare Gerät ein Herzschrittmacher, ein Kardioverter, ein Defibrillator oder eine Kombination aus diesen ist.

## Claims

1. A contact arrangement (101, 201) for connecting an electrode lead to an implantable device, comprising
an electric connection socket having a socket longitudinal axis (114, 214) and at least one opening for inserting a plug along the socket longitudinal axis (214),
comprising at least one electrically conductive spring contact element (107, 108, 230, 231, 232, 233, 234, 235) which is designed and located such that it can move outwardly in a resiliently elastic manner when a plug is inserted, become compressed at least in a spring deflection direction extending transversely to the socket longitudinal axis, and develop a spring force which counteracts the compression, wherein
the spring contact element (107, 108, 230, 231, 232, 233, 234, 235) comprises a one-piece, metallic spring contact tab having spring contact tab sections extending transversely to the spring deflection direction (120, 122), which are interconnected by way of turned sections, wherein turned sections located directly one after the other in the spring deflection direction (120, 122) have mutually opposed turning directions, **characterized in that** the spring contact element has three or four turned sections.

2. The contact arrangement (101, 201) according to claim 1, **characterized in that** the spring contact tab is turned such that the spring contact tab has a wave shape as viewed on a plane spanned by the socket longitudinal axis (114, 214) and the spring deflection direction (120, 122).

3. The contact arrangement (101, 201) according to claim 2, **characterized in that** the wave shape is an S-shaped or rectangular or triangular wave shape.

4. The contact arrangement (101, 201) according to one of the preceding claims, **characterized in that** the spring contact tab of the spring contact element (107, 108, 230, 231, 232, 233, 234, 235) is a turned leaf spring having a leaf spring cross-sectional profile, wherein the width dimension of the leaf spring cross-sectional profile is greater than the height dimension of the leaf spring cross-sectional profile.

5. The contact arrangement (101, 201) according to one of the preceding claims, which comprises at least three spring contact elements (107, 108, 230, 231, 232, 233, 234, 235), wherein the spring deflection directions of the spring contact elements (107, 108, 230, 231, 232, 233, 234, 235) are disposed on a common plane extending perpendicularly to the socket longitudinal axis (114, 214).

6. The contact arrangement (101, 201) according to one of the preceding claims, **characterized in that** a contact section of the spring contact tab comprises a convexly shaped surface region provided for contacting a countercontact of an electrode lead plug.

7. The contact arrangement (101, 201) according to one of the preceding claims, **characterized in that** the spring contact element (107, 108, 230, 231, 232, 233, 234, 235) comprises at least one elastomer element which at least partially fills an intermediate space extending between two leaf spring sections located opposite one another in the radial direction, wherein the elastomer element is configured and disposed as a deflection travel limiting element.

8. The contact arrangement (101, 201) according to one of the preceding claims, **characterized in that** the arrangement comprises a sleeve (105) having a sleeve longitudinal axis (114, 214) and a sleeve wall (106), wherein
the sleeve wall (106) encloses a sleeve interior space (104) which has at least one opening for receiving an electrode plug in the direction of the sleeve longitudinal axis (114, 214),
and wherein the sleeve longitudinal axis (114, 214) and the socket longitudinal axis (114, 214) are parallel to one another, and the spring contact element (107, 108, 230, 231, 232, 233, 234, 235) is disposed at least in part in the sleeve interior space (104).

9. A spring contact element arrangement for a contact arrangement according to one of the preceding claims, comprising a spring contact element carrier (215) which has at least three integrally formed spring contact elements (230, 231, 232, 233, 234, 235), wherein the spring contact element carrier (215) is in the form of a circular flat disk which has an opening for passage of a plug.

10. The spring contact element arrangement according to claim 9, **characterized in that** the spring contact elements (230, 231, 232, 233, 234, 235) are located equidistantly in the circumferential direction on the spring contact element carrier (215) and are integrally formed in the region of the outer edge of the spring contact element carrier (215).

11. The spring contact element arrangement according to claim 9 or 10, **characterized in that** the spring contact elements (230, 231, 232, 233, 234, 235) are each formed by a leaf spring tab turned in a wave shape, wherein the leaf spring tab has two turned sections which turn in mutually opposed turning directions and therefore has an S-shaped wave shape, and the leaf spring tabs are designed to develop a spring force, upon deflection along the spring deflection axis, which counteracts the deflection, and to move outwardly in an elastically resilient manner upon insertion of a plug.

12. The spring contact element arrangement according to one of the claims 9 to 11, **characterized in that** the spring contact element carrier (215) has recess regions adjoining each of the spring contact elements laterally in the circumferential direction.

13. The spring contact element arrangement according to one of the claims 9 to 12 **characterized in that** the spring contact element carrier (215) comprises recess regions, one of which is opposite every spring contact element, in the circumferential direction on the inner edge which adjoins the passage through the spring contact element carrier.

14. The spring contact element arrangement according to one of the claims 9 to 13, **characterized in that** the spring contact element carrier (215) comprising the spring contact elements is made of a single sheet metal piece which has leaf spring strips pointing radially outwardly in the manner of rays, and each of the spring contact elements is formed by the leaf spring strips pointing radially outwardly in the manner of rays, wherein the leaf spring strip projections on the circumference of the spring contact element carrier (215) are angled and, in the further extension thereof, have an S-shaped wave shape.

15. The spring contact element arrangement according to claim 14, **characterized in that** the leaf spring strips on the circumferential projection of the spring contact element carrier (215) are angled at a right angle.

16. The spring contact element arrangement according to one of the claims 14 or 15, **characterized in that** the recesses are cut at the side of the spring contact elements in the radial direction to such a depth that, after at least one right-angled turn of a leaf spring strip, one section of the leaf spring strip starting on the circumference of the spring contact element carrier (215) does not exceed an outer circumferential radius of the spring contact element carrier (215) next to the recesses.

17. An implantable device comprising a contact arrangement (101, 201) or a spring contact element arrangement according to one of the preceding claims, **characterized in that** the implantable device is a cardiac pacemaker, a cardioverter, a defibrillator or a combination thereof.

## Revendications

1. Dispositif de contact (101, 201) pour le raccordement d'une ligne d'électrode à un appareil implantable, avec
- une douille de raccordement électrique avec un axe longitudinal de douille (114, 214) et au moins une ouverture pour l'introduction d'un connecteur le long de l'axe longitudinal de douille (214),
- au moins un élément de contact à ressort (107, 108, 230, 231, 232, 233, 234, 235) électriquement conducteur, lequel est conçu et agencé pour s'écarter vers l'extérieur par élasticité de ressort lors de l'introduction d'un connecteur, tout en étant comprimé dans au moins une direction de déviation de ressort s'étendant transversalement à l'axe longitudinal de la douille, et en formant une force de ressort agissant contre la compression, dans lequel
- l'élément de contact à ressort (107, 108, 230, 231, 232, 233, 234, 235) comporte une langue de contact à ressort métallique monobloc, avec des sections de langue de contact à ressort s'étendant transversalement à la direction de déviation de ressort (120, 122), reliées entre elles par des sections tournantes, où les sections tournantes immédiatement consécutives dans la direction de déviation de ressort (120, 122) présentent chacune un sens d'orientation inverse,
**caractérisé en ce que** l'élément de contact à ressort comporte trois ou quatre sections tournantes.

2. Dispositif de contact (101, 201) selon la revendication 1, **caractérisé en ce que** la langue de contact à ressort est tournée de telle manière que la langue de contact à ressort présente une forme d'onde dans une vue d'au-dessus sur un plan s'étendant à partir de l'axe longitudinal de douille (114, 214) et de la direction de déviation de ressort (120, 122).

3. Dispositif de contact (101, 201) selon la revendication 2, **caractérisé en ce que** la forme d'onde est une forme d'onde en S ou encore une forme d'onde rectangulaire ou triangulaire.

4. Dispositif de contact (101, 201) selon l'une des revendications précédentes, **caractérisé en ce que** la langue de contact à ressort de l'élément de contact à ressort (107, 108, 230, 231, 232, 233, 234, 235) est un ressort à lames tourné avec un profil de section transversale de ressort à lames, dans lequel la dimension en largeur du profil de section transversale du ressort à lames est plus grande que la dimension en hauteur du profil de section transversale du ressort à lames.

5. Dispositif de contact (101, 201) selon l'une des revendications précédentes, comportant au moins trois éléments de contact à ressort (107, 108, 230, 231, 232, 233, 234, 235), dans lequel les directions de déviation de ressort des éléments de contact à ressort (107, 108, 230, 231, 232, 233, 234, 235) sont agencées sur un même plan perpendiculaire à l'axe longitudinal de douille (114, 214).

6. Dispositif de contact (101, 201) selon l'une des revendications précédentes, **caractérisé en ce qu'**une section de contact de la langue de contact à ressort comporte une zone de surface formée de façon convexe, pour le contact avec un contre-contact d'un connecteur de ligne d'électrode.

7. Dispositif de contact (101, 201) selon l'une des revendications précédentes, **caractérisé en ce que** l'élément de contact à ressort (107, 108, 230, 231, 232, 233, 234, 235) comporte au moins un élément élastomère remplissant au moins partiellement un espace intermédiaire s'étendant entre deux sections de ressort à lames opposées dans le sens radial, où l'élément élastomère est conçu et agencé comme une délimitation du trajet de déviation.

8. Dispositif de contact (101, 201) selon l'une des revendications précédentes, **caractérisé en ce que** le dispositif comporte une cosse (105) avec un axe longitudinal de cosse (114, 214) et une paroi de cosse (106), dans lequel
la paroi de cosse (106) entoure un espace intérieur de cosse (104) présentant au moins une ouverture pour l'admission d'un connecteur d'électrode dans la direction de l'axe longitudinal de cosse (114, 214),
et dans lequel l'axe longitudinal de cosse (114, 214) et l'axe longitudinal de douille (114, 214) sont parallèles entre eux, et l'élément de contact à ressort (107, 108, 230, 231, 232, 233, 234, 235) est agencé au moins partiellement dans l'espace intérieur de cosse (104).

9. Bloc d'élément de contact à ressort pour dispositif de contact selon l'une des revendications précédentes, avec un support d'élément de contact à ressort (215) comportant au moins trois éléments de contact à ressort (230, 231, 232, 233, 234, 235), dans lequel le support d'élément de contact à ressort (215) est conçu comme un disque plat circulaire comportant une interruption pour l'insertion d'un connecteur.

10. Bloc d'élément de contact à ressort selon la revendication 9, **caractérisé en ce que** les éléments de contact à ressort (230, 231, 232, 233, 234, 235) sont agencés de façon régulière dans le sens du pourtour sur le support d'élément de contact à ressort (215), et formés dans la région du bord extérieur du support d'élément de contact à ressort (215).

11. Bloc d'élément de contact à ressort selon la revendication 9 ou 10, **caractérisé en ce que** les éléments de contact à ressort (230, 231, 232, 233, 234, 235) sont constitués chacun d'une langue à ressort à lames en forme d'onde, dans lequel la langue à ressort à lames comporte deux sections tournantes présentant chacune un sens d'orientation inverse, donnant ainsi une forme d'onde en S, et les langues à ressort à lame sont conçues de manière à produire une force de ressort agissant contre une déviation, lors de la déviation le long de l'axe de déviation du ressort, tout en s'écartant vers l'extérieur par élasticité de ressort lors de l'insertion d'un connecteur.

12. Bloc d'élément de contact à ressort selon l'une des revendications 9 à 11, **caractérisé en ce que** le support d'élément de contact à ressort (215) comporte des régions échancrées faisant respectivement suite latéralement aux éléments de contact à ressort.

13. Bloc d'élément de contact à ressort selon l'une des revendications 9 à 12, **caractérisé en ce que** le support d'élément de contact à ressort (215) comporte des régions échancrées situées respectivement en face d'un élément de contact à ressort, dans le sens du pourtour, sur le bord intérieur adjacent à l'interruption du support d'élément de contact à ressort.

14. Bloc d'élément de contact à ressort selon l'une des revendications 9 à 13, **caractérisé en ce que** le support d'élément de contact à ressort (215) est formé intégralement avec les éléments de contact à ressort, à partir d'une tôle comportant des bandes de ressort à lames orientées radialement vers l'extérieur, et les éléments de contact à ressort sont constitués respectivement des bandes de ressort à lames orientées radialement vers l'extérieur, dans lequel les commencements des bandes de ressort à lames sont coudés sur le pourtour du support d'élément de contact à ressort (215) et se poursuivent par une forme d'onde en S.

15. Bloc d'élément de contact à ressort selon la revendication 14, **caractérisé en ce que** les bandes de ressort à lames sont coudées en angle droit au commencement du pourtour du support d'élément de contact à ressort (215).

16. Bloc d'élément de contact à ressort selon l'une des revendications 14 à 15, **caractérisé en ce que** les échancrures sur le côté des élément de contact à ressort sont découpées dans le sens radial avec une profondeur telle, que suite à au moins un coude en angle droit d'une bande de ressort à lames, une section de la bande de ressort à lames commençant sur le pourtour du support d'élément de contact à ressort (215) ne dépasse pas un rayon de pourtour extérieur du support d'élément de contact à ressort (215) à côté des échancrures.

17. Appareil implantable avec un dispositif de contact (101, 201) ou un bloc d'élément de contact à ressort selon l'une des revendications précédentes, **caractérisé en ce que** l'appareil implantable est un stimulateur cardiaque, un cardioverteur, un défibrillateur ou une combinaison de ceux-ci.
